# FASCICULE DE BREVET EUROPEEN

(11) **EP 1 927 376 B1**
(45) Date de publication et mention de la délivrance du brevet: **11.01.2012**
(21) Numéro de dépôt: 07121663.4
(22) Date de dépôt: 27.11.2007
(51) Int. Cl.: A61Q 5/10, A61K 8/49

(54) **Composition de coloration de pH acide comprenant la 2,3-diamino -6,7-dihydro- 1H,5H- pyrazolo[1,2-a]pyrazol-1-one, un coupleur, un agent tensio-actif particulier et un agent oxydant**
Saure Färbezusammensetzung enthaltend 2,3-Diamino-6,7-dihydro-1H,5H-pyrazolo[1,2-a]pyrazol-1-on, einen Kuppler, ein Tensid und Oxidationsmittel
Dye composition of acidic pH comprising 2,3-diamino-6,7-dihydro-1H,5H-pyrazolo[1,2-a]pyrazol-1-one, a coupler, a particular surfactant and an oxidizing agent

(30) Priorité: 30.11.2006 FR 0655213
(43) Date de publication de la demande: 04.06.2008
(73) Titulaire: L'Oréal, 75008 Paris (FR)
(72) Inventeur: Cottard, François, 92400, COURBEVOIE (FR); Laurent, Florence, 92270, BOIS COLOMBES (FR); Allard, Delphine, NEW-JERSEY, 07090 (US)
(74) Mandataire: Prevel, Estelle Nicole

(56) Documents cités:
- US-A1- 2001 023 514
- US-A1- 2005 166 335

## Description

L'invention a pour objet une composition pour la teinture des fibres kératiniques, et en particulier des fibres kératiniques humaines telles que les cheveux, comprenant la 2,3-diamino-6,7-dihydro-1 H,5H-pyrazolo[1,2-a]pyrazol-1 -one ou l'un de ses sels d'addition à titre de base d'oxydation, au moins un coupleur, au moins un agent tensio-actif choisi parmi les alcools gras oxyalkylénés ou glycérolés, les agents tensio-actifs anioniques de type sulfate ou sulfonate et les agents tensio-actifs amphotères de type bétaïne, et au moins un agent oxydant, le pH de la composition allant de 5,5 à 7,5.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou paraphénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation classiques telles que le paraaminophénol, l'orthoaminophénol et leurs dérivés éventuellement associées à des coupleurs classiques à pH acide ne permettent pas d'obtenir des nuances reflets dans les tons rouge, cuivré ou acajou qui soient suffisamment visibles sur cheveux naturels ou colorés et homogènes de la racine à la pointe.

Le document US 2005/166335 décrit une composition tinctoriale des fibres kératiniques comprenant, à titre de base d'oxydation, du 2,3-diamino-6,7-dihydro-1H,5H-pyrazolo [1,2-a]-pyrazol-1-one, un coupleur, un agent 7. tensio-actif (C₈-C₁₀ alkyl polyglucoside), un agent oxydant; le pH de la composition est 7.

Le but de la présente invention est de fournir de nouvelles compositions de coloration des fibres kératiniques qui permettent d'obtenir à pH acide une coloration aux reflets dans les tons rouge, cuivré ou acajou particulièrement visibles, puissante, chromatique, esthétique, peu sélective et résistant bien aux diverses agressions que peuvent subir les cheveux tels que les shampooings, la lumière, la sueur et les déformations permanentes.

La présente invention a donc pour objet une composition de coloration des fibres kératiniques comprenant, dans un milieu approprié pour la teinture :
- au moins une base d'oxydation choisie parmi la 2,3-diamino-6,7-dihydro-1H,5H-pyrazolo[1,2-a]pyrazol-1-one de formule (I) suivante et ses sels d'addition : 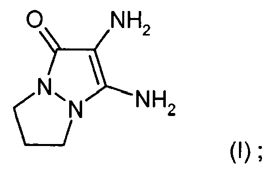
- au moins un coupleur ;
- au moins un agent tensio-actif choisi parmi les alcools gras oxyalkylénés ou glycérolés, les agents tensio-actifs anioniques de type sulfate ou sulfonate et les agents tensio-actifs amphotères de type bétaïne ; et
- au moins un agent oxydant ;
le pH de la composition allant de 5,5 à 7,5 ;
avec la condition que lorsque la composition comprend un alcool gras oxyalkyléné ou glycérolé, elle est exempte de 3-amino 2-méthylamino 6-méthoxy pyridine ; de polyuréthane cationique à chaîne grasse obtenu à partir de la condensation de 1,3-bis-(isocyanatométhylcyclohexane), de N,N-diméthyléthanolamine quaternisée avec le bromodécane, de N,N-diméthyléthanolamine et de polyoxyéthylène ; de polycondensat de polyéthylèneglycol à 150 ou 180 moles d'oxyde d'éthylène, d'alcool décylique et de méthylène-bis(4-cyclohexylisocyanate) ; des mélanges d'alcool cétéarylique polyglycérolé à 2 moles de glycérol et d'alcool cétéarylique polyglycérolé à 6 moles de glycérol ; d'hexylèneglycol.

La présente invention permet d'obtenir une coloration des fibres kératiniques aux reflets dans les tons rouge, cuivré ou acajou qui soient suffisamment visibles sur cheveux naturels ou colorés et homogènes de la racine à la pointe.

Un autre objet de l'invention est un procédé de teinture des fibres kératiniques, et en particulier des fibres kératiniques humaines telles que les cheveux, mettant en oeuvre la composition de la présente invention, ainsi que l'utilisation de cette composition pour la teinture des fibres kératiniques.

L'invention a enfin pour objet un kit de coloration comprenant d'une part une composition de coloration contenant la 2,3-diamino-6,7-dihydro-1H,5H-pyrazolo[1,2-a]pyrazol-1-one ou l'un de ses sels d'addition à titre de base d'oxydation, un coupleur et un agent tensio-actif choisi parmi les alcools gras oxyalkylénés ou glycérolés, les agents tensio-actifs anioniques de type sulfate ou sulfonate et les agents tensio-actifs amphotères de type bétaïne et d'autre part une composition contenant un agent oxydant.

A moins d'une indication différente, les bornes des gammes de valeurs qui sont données dans le cadre de la présente invention sont incluses dans ces gammes.

La ou les bases d'oxydation choisies parmi la 2,3-diamino-6,7-dihydro-1H,5H-pyrazolo[1,2-a]pyrazol-1-one de formule (I) et ses sels d'addition sont en général présentes dans la composition de l'invention chacune en quantité comprise entre 0,001 à 10 % en poids environ du poids total de la composition tinctoriale, de préférence entre 0,005 et 6 %.

Le ou les coupleurs utiles dans le cadre de l'invention peuvent être choisis parmi les méta-phénylènediamines, les méta-aminophénols, les méta-diphénols, les coupleurs naphtaléniques, les coupleurs hétérocycliques et leurs sels d'addition.

A titre d'exemples, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Selon un mode de réalisation préféré de l'invention, le ou les coupleurs sont choisis parmi les méta-aminophénols. De préférence, les méta-aminophénols utiles dans le cadre de l'invention sont choisis parmi les composés de formule (II) suivante et leurs sels d'addition : 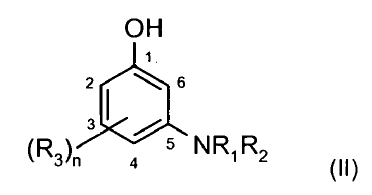 dans laquelle :
R₁ et R₂, identiques ou différents, représentent un atome d'hydrogène ; un radical alkyle ; un radical monohydroxyalkyle ; un radical polyhydroxyalkyle ; un radical monoaminoalkyle ;
R₁ et R₂ peuvent également former entre eux et avec l'atome d'azote auquel ils sont reliés un groupement cyclique contenant un ou plusieurs hétéroatomes, de 5 à 7 chaînons, saturé ou insaturé, non substitué ou substitué par un ou plusieurs radicaux choisis parmi les radicaux carboxy, carboxamido, hydroxyle, amino, mono ou dialkylamino, alkyle éventuellement substitué par un ou plusieurs radicaux hydroxyle, amino, mono ou dialkylamino ;
R₃ représentent, indépendamment les uns des autres, un atome d'halogène ; un radical alkyle ; un radical alcoxy ; un radical monohydroxyalkyle ; un radical polyhydroxyalkyle ; un radical monohydroxyalcoxy ; un radical polyhydroxyalcoxy ;
n est un entier compris entre 0 et 3.

Dans le cadre de l'invention, on entend par radical alkyle des radicaux alkyle linéaires ou ramifiés en C₁-C₁₀ sauf indication contraire, préférentiellement en C₁-C₆, encore plus préférentiellement en C₁-C₄, tels que le radical méthyle, éthyle, propyle, isopropyle, isobutyle, tertiobutyle, pentyle, hexyle.

Dans le cadre de la présente invention, le ou les hétéroatomes peuvent être choisis parmi un atome d'oxygène, un atome d'azote, un atome de soufre, un atome de phosphore.

Dans le cadre de la présente invention, un atome d'halogène peut être choisi parmi un atome de chlore, un atome de brome, un atome d'iode et un atome de fluor.

Selon un mode de réalisation particulier de l'invention, R₁ et R₂ représentent, indépendamment l'un de l'autre, un atome d'hydrogène ; un radical alkyle par exemple un radical méthyle ou éthyle ; un radical monohydroxyalkyle par exemple un radical β-hydroxyéthyle ou γ-hydroxypropyle ; ou R₁ et R₂ forment entre eux et avec l'atome d'azote auquel ils sont reliés un cycle choisi parmi les hétérocycles pyrrolidine, pipéridine, homopipéridine, pipérazine, homopipérazine, morpholine ; lesdits cycles pouvant être substitués par un ou plusieurs radicaux hydroxyle, amino, mono ou dialkyl(C₁-C₂)amino, carboxy, carboxamido, alkyle en C₁-C₄ éventuellement substitué par un ou plusieurs radicaux hydroxyle, amino, mono ou dialkyl(C₁-C₂)amino, et plus particulièrement choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(β-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, la 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine, la pipérazine, la 4-méthyl-pipérazine, la 4-éthyl-pipérazine, la 4-(β-hydroxyéthyl)-pipérazine, la morpholine, et plus particulièrement ils forment un groupement pyrrolidin-1-yle ; pipéridin-1-yle ; pipérazin-1-yle ; 4-méthyl-pipérazin-1-yle ; 4-éthyl-pipérazin-1-yle ; 4-(β-hydroxyéthyl)-pipérazin-1-yle ; morpholin-4-yle.

Selon un mode de réalisation particulier de l'invention, R₃ est choisi parmi un atome d'halogène, un radical alkyle, un radical alcoxy, un radical monohydroxyalcoxy. A titre d'exemple, R₃ est choisi parmi un atome de chlore, un radical méthyle, un radical méthoxy, un radical β-hydroxyéthyloxy.

Selon un mode de réalisation particulier de l'invention, n désigne 0, 1 ou 2. A titre d'exemple, n est égal à 1 ou 2. Lorsque n est égal à 1, R₃ peut se situer en position 2 ou 6 et lorsque n est égal à 2, R₃ peuvent se situer en positions 2 et 4 ou en position 2 et 6.

Parmi les méta-aminophénols substitués de formule (II) utiles dans le cadre de l'invention, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 6-chloro 2-méthyl 5-amino phénol, le 5-amino 2,4-diméthoxy phénol, le 5-(γ-hydroxypropylamino) 2-méthyl phénol, le 3-diméthylamino-phénol ; le 2-méthyl-5-diméthylamino-phénol ; le 2-éthyl-5-diméthylamino-phénol ; le 2-méthoxy-5-diméthylamino-phénol ; le 2-éthoxy-5-diméthylamino-phénol ; le 2-(β-hydroxyéthyl)-5-diméthylamino-phénol ; le 3-diéthylamino-phénol ; le 2-méthyl-5-diéthylamino-phénol ; le 2-éthyl-5-diéthylamino-phénol ; le 2-méthoxy-5-diéthylamino-phénol ; le 2-éthoxy-5-diéthylamino-phénol ; le 2-(β-hydroxyéthyl)-5-diéthylamino-phénol ; le 3-di(β-hydroxyéthyl)amino-phénol ; le 2-méthyl-5-di(β-hydroxyéthyl)amino-phénol ; le 2-éthyl-5-di(β-hydroxyéthyl)amino-phénol ; le 2-méthoxy-5-di(β-hydroxyéthyl)amino-phénol ; le 2-éthoxy-5-di(β-hydroxyéthyl)amino-phénol ; le 2-(β-hydroxyéthyl)-5-di(β-hydroxyéthyl)amino-phénol ; le 3-pyrrolidin-1-yl-phénol ; le 2-méthyl-5-pyrrolidin-1-yl-phénol ; le 2-éthyl-5-pyrrolidin-1-yl-phénol ; le 2-méthoxy-5-pyrrolidin-1-yl-phénol ; le 2-éthoxy-5-pyrrolidin-1-yl-phénol ; le 2-(β-hydroxyéthyl)-5-pyrrolidin-1-yl-phénol ; le 3-pipéridin-1-yl-phénol ; le 2-méthyl-5-pipéridin-1-yl-phénol ; le 2-éthyl-5-pipéridin-1-yl-phénol ; le 2-méthoxy-5-pipéridin-1-yl-phénol ; le 2-éthoxy-5-pipéridin-1-yl-phénol ; le 2-(β-hydroxyéthyl)-5-pipéridin-1-yl-phénol ; le 3-pipérazin-1-yl-phénol ; le 2-méthyl-5-pipérazin-1-yl-phénol ; le 2-éthyl-5-pipérazin-1-yl-phénol ; le 2-méthoxy-5-pipérazin-1-yl-phénol ; le 2-éthoxy-5-pipérazin-1-yl-phénol ; le 2-(β-hydroxyéthyl)-5-pipérazin-1-yl-phénol ; le 3-(4-méthyl-pipérazin-1-yl)-phénol ; le 2-méthyl-5-(4-méthyl-pipérazin-1-yl)-phénol ; le 2-éthyl-5-(4-méthyl-pipérazin-1-yl)-phénol ; le 2-méthoxy-5-(4-méthyl-pipérazin-1-yl)-phénol ; le 2-éthoxy-5-(4-méthyl-pipérazin-1-yl)-phénol ; le 2-(β-hydroxyéthyl)-5-(4-méthyl-pipérazin-1-yl)-phénol ; le 3-(4-éthyl-pipérazin-1-yl)-phénol ; le 2-méthyl-5-(4-éthyl-pipérazin-1-yl)-phénol ; le 2-éthyl-5-(4-éthyl-pipérazin-1-yl)-phénol ; le 2-méthoxy-5-(4-éthyl-pipérazin-1-yl)-phénol ; le 2-éthoxy-5-(4-éthyl-pipérazin-1-yl)-phénol ; le 2-(β-hydroxyéthyl)-5-(4-éthyl-pipérazin-1-yl)-phénol ; le 3-(4-(β-hydroxyéthyl)-pipérazin-1-yl)-phénol ; le 2-méthyl-5-(4-(β-hydroxyéthyl)-pipérazin-1-yl)-phénol ; le 2-éthyl-5-(4-(β-hydroxyéthyl)-pipérazin-1-yl)-phénol ; le 2-méthoxy-5-(4-(β-hydroxyéthyl)-pipérazin-1-yl)-phénol ; le 2-éthoxy-5-(4-(β-hydroxyéthyl)-pipérazin-1-yl)-phénol ; le 2-(β-hydroxyéthyl)-5-(4-(β-hydroxyéthyl)-pipérazin-1-yl)-phénol ; le 3-morpholin-4-yl-phénol ; le 2-méthyl-5-morpholin-4-yl-phénol ; le 2-éthyl-5-morpholin-4-yl-phénol ; le 2-méthoxy-5-morpholin-4-yl-phénol ; le 2-éthoxy-5-morpholin-4-yl-phénol ; le 2-(β-hydroxyéthyl)-5-morpholin-4-yl-phénol.

Selon un mode de réalisation préféré, R₁ et R₂ désignent indépendemment l'un de l'autre un atome d'hydrogène ou un radical mono ou polyhydroxyalkyle. Le 5-amino 2-méthyl phénol et le 5-[N-(β-hydroxyéthyl)amino] 2-méthyl phénol sont particulièrement préférés.

Selon un autre mode de réalisation préféré, le ou les méta-aminophénols sont choisis parmi les méta-aminophénols chlorés. Dans le sens de la présente invention, on entend par méta-aminophénol chloré un méta-aminophénol comprenant dans sa structure au moins un atome de chlore. Le 6-chloro 2-méthyl 5-amino phénol est particulièrement préféré.

Dans la composition de la présente invention, le ou les coupleurs sont en général chacun présents en quantité comprise entre 0,001 et 10 % en poids environ du poids total de la composition tinctoriale, de préférence entre 0,005 et 6 %.

Le ou les alcools gras oxyalkylénés peuvent être choisis parmi les composés de formule (III) suivante : 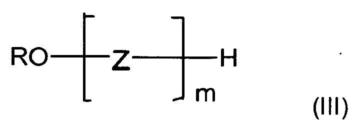 dans laquelle :
R désigne un radical saturé ou insaturé, linéaire ou ramifié, comportant de 8 à 40 atomes de carbone et de préférence de 8 à 30 ;
Z représente un radical oxyéthyléné (i) et / ou oxypropyléné (ii)₁ et (ii)₂ de formules respectives suivantes :

   -CH₂-CH₂-O- (i)

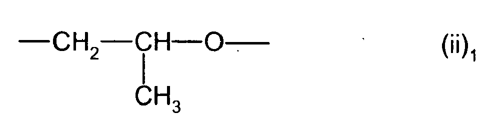

   -CH₂-CH₂-CH₂-O- (ii)₂.
m représente le nombre de groupements oxyde d'éthylène (i) et / ou oxyde de propylène (ii)₁ ou (ii)₂ , allant de 1 et 250 et de préférence de 2 à 100.

Le ou les alcools gras glycérolés peuvent être choisis parmi les composés de formule (IV) suivante : 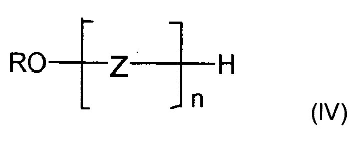 dans laquelle :
R désigne un radical saturé ou insaturé, linéaire ou ramifié, comportant de 8 à 40 atomes de carbone et de préférence de 8 à 30 ;
Z représente un radical glycérolé (iii) de formule suivante : 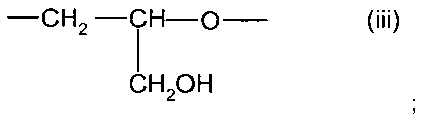
n représente le nombre de groupements glycérol (iii) et est compris entre 1 et 30 et de préférence entre 1 et 10.

La composition selon l'invention peut renfermer des mélanges de ces alcools gras oxyalkylénés ou glycérolés.

Des alcools gras oxyalkylénés particulièrement préférés selon l'invention sont des alcools gras saturés ou non, linéaires ou ramifiés, comportant de 10 à 20 atomes de carbone et 2 à 40 groupements oxyde d'éthylène.

Comme composés de type alcool gras oxyalkyléné, on peut notamment citer les produits commercialisés suivants :
Mergital LM2 (COGNIS) [alcool laurique 2 OE ] ;
Ifralan L12 (IFRACHEM) et Rewopal 12 (GOLDSCHMITDT) [alcool laurique 12 OE] ;
Empilan KA 2.5/90FL (ALBRIGHT & WILSON) et Mergital BL309 (COGNIS) [alcool décylique 3 OE] ;
Empilan KA 5/90FL (ALBRIGHT & WILSON) et Mergital BL589 (COGNIS) [alcool décylique 5 OE] ;
Brij 58 (UNIQUEMA) et Simulsol 58 (SEPPIC) [alcool cétylique 20 OE] ;
Eumulgin 05 (COGNIS) [alcool oleocétylique 5 OE] ;
Mergital OC30 (COGNIS) [alcool oleocétylique 30 OE] ;
Brij 72 (UNIQUEMA) [alcool stéarylique 2 OE] ;
Brij 76 (UNIQUEMA) [alcool stéarylique 10 OE] ;
Brij 78P (UNIQUEMA) [alcool stéarylique 20 OE] ;
Brij 700 (UNIQUEMA) [alcool stéarylique 100 OE];
Eumulgin B1 (COGNIS) [alcool cétylstéarylique 12 OE] ;
Eumulgin L (COGNIS) [alcool cétylique 9 OE et 2 OP] ;
Witconol APM (GOLDSCHMIDT) [alcool myristique 3 OP].

Comme composés de type alcool gras glycérolé, on peut notamment citer l'alcool laurique à 4 moles de glycérol (nom INCl : POLYGLYCERYL-4 LAURYL ETHER), l'alcool oléique à 4 moles de glycérol (nom INCl : POLYGLYCERYL-4 OLEYL ETHER), l'alcool oléique à 2 moles de glycérol (nom INCI : POLYGLYCERYL-2 OLEYL ETHER), l'alcool cétéarylique à 2 moles de glycérol, l'alcool cétéarylique à 6 moles de glycérol , l'alcool oléocétylique à 6 moles de glycérol, l'octadécanol à 6 moles de glycerol, et l'alcool laurique à 1,5 moles de glycerol (nom INCl : GLYCERYL LAURYL ETHER).

L'alcool gras peut représenter un mélange d'alcools gras, ce qui signifie que dans un produit commercial peuvent coexister plusieurs espèces d'alcools gras oxyalkylénés ou glycérolés sous forme d'un mélange.

Selon l'invention, les agents tensio-actifs anioniques de type sulfate ou sulfonate sont des agents tensio-actifs anioniques comportant au moins une fonction sulfate (-OSO₃H ou -OSO₃-) et / ou une fonction sulfonate (-SO₃H ou -SO₃-).

Les agents tensio-actifs anioniques de type sulfate ou sulfonate utilisables, seuls ou mélanges, dans le cadre de la présente invention, sont par exemple les sels (en particulier sels alcalins, notamment de sodium, sels d'ammonium, sels d'amines, sels d'aminoalcools ou sels de magnésium) des alkylsulfates, des alkylamidosulfates, des alkyléthersulfates, des alkylamidoéthersulfates, des alkylaryléthersulfates, des alkyléthersulfosuccinates, des acyl iséthionates, des méthyl acyl taurates, des α-oléfines sulfonates ; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Le ou les agents tensio-actifs amphotères de type bétaïne peuvent être choisis parmi les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines, en particulier la cocamidopropylbétaine telles que la TEGOBETAINE^{®} F50 commercialisée par la société GOLDSCHMIDT.

Le ou les agents tensio-actifs choisi parmi les alcools gras oxyalkylénés ou glycérolés, les agents tensio-actifs anioniques de type sulfate ou sulfonate et les agents tensio-actifs amphotères de type bétaïne sont généralement présents en quantité comprise entre 0,05 et 50 %, et de préférence entre 0,5 et 40 %, et encore plus préférentiellement entre 1 et 20 % en poids du poids total de la composition.

La composition de la présente invention peut comprendre au moins une base d'oxydation additionnelle choisie parmi les para-phénylènediamines, les bis-phénylalkylènediamines, les para-aminophénols, les bis-para-aminophénols, les ortho-aminophénols, les ortho-phénylènediamines, les bases hétérocycliques différentes de la 2,3-diamino-6,7-dihydro-1H,5H-pyrazolo[1,2-a]pyrazol-1-one et ses sels d'addition.

Parmi les para-phénylènediamines, on peut citer, à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine, la 6-(4-amino-phénylamino)-hexan-1-ol et leurs sels d'addition.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, la 6-(4-amino-phénylamino)-hexan-1-ol et leurs sels d'addition sont particulièrement préférées.

Parmi les bis-phénylalkylènediamines, on peut citer, à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer, à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer, à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1 ,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Lorsque la composition de l'invention comprend une ou plusieurs bases d'oxydation additionnelles, celles-ci sont en général présentes chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les alkyl(C₁-C₄)sulfonates, les tosylates, les benzènesulfonates, les phosphates et les acétates et les sels d'addition avec une base telles que la soude, la potasse, l'ammoniaque, les amines ou les alcanolamines.

Le ou les agents oxydants peuvent être choisis parmi les agents oxydants classiquement utilisés pour la teinture d'oxydation des fibres kératiniques comme par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydoréductases à 2 électrons telles que les uricases et les oxygénases à 4 électrons comme les laccases, les enzymes oxydases étant éventuellement en présence de leurs cofacteurs. Le peroxyde d'hydrogène est particulièrement préféré.

Le ou les agents oxydants sont généralement présents en quantité comprise entre 0,01 et 30 %, de préférence entre 0,1 et 20 % en poids par rapport au poids total de la composition.

Le pH de la composition tinctoriale conforme à l'invention va de 5,5 à 7,5, et de préférence de 5,7 à 6,9. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 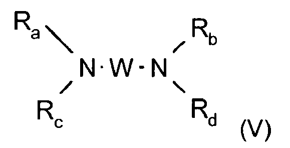 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymériques anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé conforme à la présente invention est un procédé dans lequel on applique sur les fibres la composition selon l'invention telle que définie précédemment pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 2 et 60 minutes, de préférence entre 3 et 40 minutes, et encore plus préférentiellement entre 5 et 30 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une base d'oxydation choisie parmi la 2,3-diamino-6,7-dihydro-1H,5H-pyrazolo[1,2-a]pyrazol-1-one et ses sels d'addition, au moins un coupleur et au moins un agent tensio-actif choisi parmi les alcools gras oxyalkylénés ou glycérolés, les agents tensio-actifs anioniques de type sulfate ou sulfonate et les agents tensio-actifs amphotères de type bétaïne et d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

Les compositions (A) et (B) sont telles que lorsque leur mélange comprend un alcool gras oxyalkyléné ou glycérolé, il est exempte de 3-amino 2-méthylamino 6-méthoxy pyridine ; de polyuréthane cationique à chaîne grasse obtenu à partir de la condensation de 1,3-bis-(isocyanatométhylcyclohexane), de N,N-diméthyléthanolamine quaternisée avec le bromodécane, de N,N-diméthyléthanolamine et de polyoxyéthylène ; de polycondensat de polyéthylèneglycol à 150 ou 180 moles d'oxyde d'éthylène, d'alcool décylique et de méthylène-bis(4-cyclohexylisocyanate); des mélanges d'alcool cétéarylique polyglycérolé à 2 moles de glycérol et d'alcool cétéarylique polyglycérolé à 6 moles de glycérol ; d'hexylèneglycol.

Les compositions (A) et (B) peuvent également renfermer divers adjuvants utilisés classiquement dans les compositions pour la teinture des cheveux et tels que définis précédemment.

Le pH de la composition (A) est généralement compris entre 3 et 12 environ, et de préférence entre 5 et 11 environ. Il peut être ajusté à la valeur désirée au moyen d'agents acidifiants ou alcalinisants tels que définis précédemment ou bien encore à l'aide de systèmes tampons classiques.

Le pH de la composition (B) est tel qu'après mélange avec la composition (A), le pH de la composition résultante appliquée sur les fibres kératiniques varie de 5,5 à 7,5, et encore plus préférentiellement de 5,6 à 6,9. Il peut être ajusté à la valeur désirée au moyen d'agents acidifiants ou alcalinisants tels que définis précédemment ou bien encore à l'aide de systèmes tampons classiques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

La présente invention a également pour objet l'utilisation pour la coloration d'oxydation des fibres kératiniques, et en particulier des fibres kératiniques humaines telles que les cheveux, d'une composition selon l'invention telle que définie précédemment.

Les exemples qui suivent servent à illustrer l'invention sans toutefois présenter un caractère limitatif.

### EXEMPLES

### Exemple 1

La composition suivante a été réalisée :

| | |
|---|---|
| Séquestrants | 2 g |
| Réducteurs | 0,71 g |
| Ethanolamine | 2,27 g |
| Acide citrique | 0,15 g |
| Silice pyrogénée à caractère hydrophobe | 1,2 g |
| 2,3-diamino-6,7-dihydro-1H,5H-pyrazolo[1,2-a]pyrazol-1-one, 2 CH₃-SO₃H | 1,9 g |
| 4-amino 2-hydroxy toluène | 0,2 g |
| 6-chloro 2-méthyl 5-amino phénol | 0,8 g |
| Para-aminophénol | 0,1 g |
| Distéarate de glycol | 2 g |
| Parfum | 0,95 g |
| Polycondensat tétraméthyl hexaméthylènediamine / dichloro 1,3-propylène | 4 g |
| Copolymère chlorure de diméthyl diallyl ammonium / acide acrylique (80/20) | 3 g |
| Carbopol 980 | 0,4 g |
| Propylène glycol | 10 g |
| Acide laurique | 3 g |
| Alcool laurique oxyéthyléné (12 OE) | 7 g |
| Alcool cétylstéarylique | 11,5 g |
| Alcool décylique oxyéthyléné (3 OE) | 10 g |
| Alcool oléocétylique oxyéthyléné (30 OE) | 4 g |
| Acide ascorbique | 0,25 g |
| Eau | 34,57 g |

Au moment de l'emploi, 1 partie en poids de la composition décrite ci-dessus est mélangée avec 1 partie en poids d'une solution de peroxyde d'hydrogène à 20 volumes dont le pH est égal à 2,3. On obtient un pH final de 6,8 +/- 0,2.
Le mélange obtenu est appliqué sur des mèches de cheveux gris à 90 % de blancs naturels ou permanentés. Après 20 minutes de pose à 22 °C +/- 3 °C, les mèches sont rincées, lavées avec un shampooing standard, rincées à nouveau puis séchées.
La coloration capillaire est évaluée de manière visuelle. On obtient une nuance avec un intense reflet cuivré.

### Exemple 2

La composition suivante a été réalisée :

| | |
|---|---|
| Séquestrants | 0,2 g |
| Antioxydants | 0,2 g |
| Ethanolamine | 0,2 g |
| Réducteurs | 1 g |
| 2,3-diamino-6,7-dihydro-1H,5H-pyrazolo[1,2-a]pyrazol-1-one, 2 CH₃-SO₃H | 1,87 g |
| 4-amino 2-hydroxy toluène | 1,96 g |
| Para-phénylènediamine | 0,77 g |
| Para-aminophénol | 0,15 g |
| Alcool oléique | 6 g |
| Acides aminés de blé | 0,5 g |
| Parfum | 0,3 g |
| Poly diméthyl / méthylsiloxane oxyéthyléné et oxypropyléné (20/5) (OE/OP 20/80) | 1,5 g |
| Alcool isopropylique | 10 g |
| Propylène glycol | 7 g |
| Monobutyl éther de dipropylène glycol | 5 g |
| Cocoyl bétaïne (solution aqueuse à 30 %) | 2,5 g |
| Phosphate acide d'alcool cétylique oxyéthyléné (10OE) et oxypropyléné (5 OP) | 0,9 g |
| Alcool décylique oxyéthyléné (3OE) | 9 g |
| Alpha oléfine sulfonate de sodium (solution aqueuse à 40 %) | 22,5 g |
| Alcool laurylique | 2,5 g |
| Monoisopropanolamide d'acides de coprah | 6,5 g |
| Eau | 19,45 g |

Au moment de l'emploi, 1 partie en poids de la composition décrite ci-dessus est mélangée avec 1 partie en poids d'une solution de peroxyde d'hydrogène à 6,7 volumes dont le pH est égal à 1,8 +/- 0,3. On obtient un pH final de 6,5 +/- 0,3.
Le mélange obtenu est appliqué sur des mèches de cheveux gris à 90 % de blancs naturels ou permanentés. Après 20 minutes de pose à 33 °C, les mèches sont rincées, lavées avec un shampooing standard, rincées à nouveau puis séchées.
La coloration capillaire est évaluée de manière visuelle. On obtient une nuance rouge.

### Exemple 3

La composition suivante a été réalisée :

| | |
|---|---|
| Séquestrants | 0,2 g |
| Antioxydants | 0,2 g |
| Ethanolamine | 0,2 g |
| Réducteurs | 1 g |
| 2,3-diamino-6,7-dihydro-1H,5H-pyrazolo[1,2-a]pyrazol-1-one, 2 CH₃-SO₃H | 0,9 g |
| 2-méthyl résorcinol | 0,2 g |
| 6-hydroxy indole | 0,7 g |
| 2-amino 3-hydroxy pyridine | 0,35 g |
| Résorcinol | 0,1 g |
| Para-phénylènediamine | 0,5 g |
| 6-chloro 2-méthyl 5-amino phénol | 0,1 g |
| Alcool oléique | 6 g |
| Acides aminés de blé | 0,5 g |
| Parfum | 0,3 g |
| Poly diméthyl / méthylsiloxane oxyéthyléné et oxypropyléné (20/5) (OE/OP 20/80) | 1,5 g |
| Alcool isopropylique | 10 g |
| Propylène glycol | 7 g |
| Monobutyl éther de dipropylène glycol | 5 g |
| Cocoyl bétaïne (solution aqueuse à 30 %) | 2,5 g |
| Phosphate acide d'alcool cétylique oxyéthyléné (10OE) et oxypropyléné (5 OP) | 0,9 g |
| Alcool décylique oxyéthyléné (3OE) | 9 g |
| Alpha oléfine sulfonate de sodium (solution aqueuse à 40 %) | 22,5 g |
| Alcool laurylique | 2,5 g |
| Monoisopropanolamide d'acides de coprah | 6,5 g |
| Eau | 21,35 g |

Au moment de l'emploi, 1 partie en poids de la composition décrite ci-dessus est mélangée avec 1 partie en poids d'une solution de peroxyde d'hydrogène à 6,7 volumes dont le pH est égal à 1,8 +/- 0,3. On obtient un pH final de 6,5 +/- 0,3.
Le mélange obtenu est appliqué sur des mèches de cheveux gris à 90 % de blancs naturels ou permanentés. Après 20 minutes de pose à 33 °C, les mèches sont rincées, lavées avec un shampooing standard, rincées à nouveau puis séchées.
La coloration capillaire est évaluée de manière visuelle. On obtient une nuance acajou.

## Revendications

1. Composition de coloration des fibres kératiniques comprenant, dans un milieu approprié pour la teinture :
• au moins une base d'oxydation choisie parmi la 2,3-diamino-6,7-dihydro-1H,5H-pyrazolo[1,2-a]pyrazol-1-one de formule (I) suivante et ses sels d'addition : 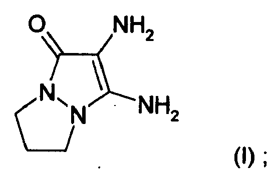
• au moins un coupleur ;
• au moins un agent tensio-actif choisi parmi les alcools gras oxyalkylénés ou glycérolés, les agents tensio-actifs anioniques de type sulfate ou sulfonate et les agents tensio-actifs amphotères de type bétaïne ; et
• au moins un agent oxydant ;
le pH de la composition allant de 5,5 à 7,5 ;
avec la condition que lorsque la composition comprend un alcool gras oxyalkyléné ou glycérolé, elle est exempte de 3-amino 2-méthylamino 6-méthoxy pyridine; de polyuréthane cationique à chaîne grasse obtenu à partir de la condensation de 1,3-bis-(isocyanatométhylcyclohexane), de N,N-diméthyléthanolamine quaternisée avec le bromodécane, de N,N-diméthytéthanolamine et de polyoxyéthylène ; de polycondensat de polyéthylèneglycol à 150 ou 180 moles d'oxyde d'éthylène, d'alcool décylique et de méthylène-bis(4-cyclohexylisocyanate) ; des mélanges d'alcool cétéarylique polyglycérolé à 2 moles de glycérol et d'alcool cétéarylique polyglycérolé à 6 moles de glycérol ; d'hexylèneglycol.

2. Composition selon la revendication 1 dans laquelle la ou les bases d'oxydation choisies parmi la 2,3-diamino-6,7-dihydro-1H,5H-pyrazolo[1,2-a]pyrazol-1-one de formule (I) et ses sels d'addition sont présentes chacune en quantité comprise entre 0,001 à 10 % en poids environ du poids total de la composition tinctoriale.

3. Composition selon la revendication 1 ou 2 dans laquelle le ou les coupleurs sont choisis parmi les méta-phénylènediamines, les méta-aminophénols, les méta-diphénols, les coupleurs naphtaléniques, les coupleurs hétérocycliques et leurs sels d'addition.

4. Composition selon la revendication 3 dans laquelle le ou les coupleurs sont choisis parmi les méta-aminophénols.

5. Composition selon la revendication 4 dans laquelle le ou les méta-aminophénols sont choisis parmi les composés de formule (II) suivante et leurs sels d'addition : 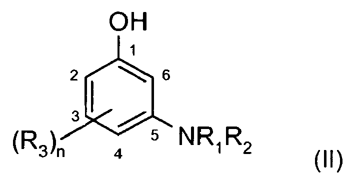 dans laquelle :
R₁ et R₂, identiques ou différents, représentent un atome d'hydrogène ; un radical alkyle ; un radical monohydroxyalkyle ; un radical polyhydroxyalkyle ; un radical monoaminoalkyle ;
R₁ et R₂ peuvent également former entre eux et avec l'atome d'azote auquel ils sont reliés un groupement cyclique contenant un ou plusieurs hétéroatomes, de 5 à 7 chaînons, saturé ou insaturé, non substitué ou substitué par un ou plusieurs radicaux choisis parmi les radicaux carboxy, carboxamido, hydroxyle, amino, mono ou
dialkylamino, alkyle éventuellement substitué par un ou plusieurs radicaux hydroxyle, amino, mono ou dialkylamino ;
R₃ représentent, indépendamment les uns des autres, un atome d'halogène ; un radical alkyle ; un radical alcoxy ; un radical monohydroxyalkyle ; un radical polyhydroxyalkyle ; un radical monohydroxyalcoxy ; un radical polyhydroxyalcoxy ;
n est un entier compris entre 0 et 3.

6. Composition selon la revendication 5 dans laquelle R₁ et R₂ désignent indépendemment l'un de l'autre un atome d'hydrogène ou un radical mono ou polyhydroxyalkyle.

7. Composition selon l'une quelconque des revendications précédentes dans laquelle le ou les coupleurs sont choisis parmi les méta-aminophénols chlorés.

8. Composition selon l'une quelconque des revendications précédentes dans laquelle le ou les coupleurs sont chacun présents en quantité comprise entre 0,001 et 10 % en poids environ du poids total de la composition tinctoriale.

9. Composition selon l'une quelconque des revendications précédentes dans laquelle le ou les alcools gras oxyalkylénés sont linéaires ou ramifiés, saturés ou insaturés, et comportent 8 à 40 atomes de carbone et 1 à 250 groupements oxyde d'éthylène et / ou oxyde de propylène.

10. Composition selon la revendication 9 dans laquelle le ou les alcools gras oxyalkylénés sont linéaires ou ramifiés, saturés ou insaturés, et comportent 10 à 20 atomes de carbone et 2 à 40 groupements oxyde d'éthylène.

11. Composition selon l'une quelconque des revendications précédentes dans laquelle le ou les alcools gras glycérolés sont linéaires ou ramifiés, saturés ou insaturés, et comportent 8 à 40 atomes de carbone et 1 à 30 groupements glycérol.

12. Composition selon l'une quelconque des revendications précédentes dans laquelle le ou les agents tensio-actifs anioniques de type sulfate ou sulfonate sont choisis parmi les sels des alkylsulfates, des alkylamidosulfates, des alkyléthersulfates, des alkylamidoéthersulfates, des alkylaryléthersulfates, des alkyléthersulfosuccinates, des acyl iséthionates, des méthyl acyl taurates, des α-oléfines sulfonates ; le radical alkyle ou acyle de tous ces différents composés comportant de 8 à 24 atomes de carbone, et le radical aryle désignant un groupement phényle ou benzyle ; le nombre moyen de groupements oxyde d'éthylène ou oxyde de propylène allant de 2 à 50.

13. Composition selon l'une quelconque des revendications précédentes, dans laquelle le ou les agents tensio-actifs amphotères de type bétaïne sont choisis parmi les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

14. Composition selon l'une quelconque des revendications précédentes dans laquelle le ou les agents tensio-actifs choisi parmi les alcools gras oxyalkylénés ou glycérolés, les agents tensio-actifs anioniques de type sulfate ou sulfonate et les agents tensio-actifs amphotères de type bétaïne représentent de 0,05 à 50 % en poids du poids total de la composition.

15. Composition selon l'une quelconque des revendications précédentes comprenant au moins une base d'oxydation additionnelle choisie parmi les paraphénylènediamines, les bis-phénylalkylènediamines, les para-aminophénols, les bispara-aminophénols, les ortho-aminophénols, les ortho-phénylènediamines, les bases hétérocycliques différentes de la 2,3-diamino-6,7-dihydro-1H,5H-pyrazolo[1,2-a]pyrazol-1-one et ses sels d'addition.

16. Composition selon l'une quelconque des revendications précédentes dans laquelle le ou les agents oxydants sont choisis parmi le peroxyde d'hydrogène, le peroxyde d'urée, les bromates de métaux alcalins, les persels, les peracides et les enzymes oxydases.

17. Composition selon l'une quelconque des revendications précédentes dans laquelle le ou les agents oxydants sont présents en quantité comprise entre 0,01 et 30 % en poids du poids total de la composition.

18. Procédé de teinture des fibres kératiniques dans lequel on applique sur les fibres la composition telle que définie à l'une quelconque des revendications 1 à 17 pendant un temps suffisant pour développer la coloration désirée, après quoi on rince, on lave éventuellement au shampooing, on rince à nouveau et on sèche.

19. Procédé selon la revendication 18 comportant une étape préliminaire consistant à stocker sous forme séparée, d'une part, une composition (A) comprenant, dans un milieu approprié pour la teinture, au moins une base d'oxydation choisie parmi la 2,3-diamino-6,7 -dihydro-1 H,5H-pyrazolo[1,2-a]pyrazol-1 -one et ses sels d'addition, au moins un coupleur et au moins un agent tensio-actif choisi parmi les alcools gras oxyalkylénés ou glycérolés, les agents tensio-actifs anioniques de type sulfate ou sulfonate et les agents tensio-actifs amphotères de type bétaïne et d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

20. Dispositif à plusieurs compartiments dans lequel un premier compartiment contient une composition (A) telle que définie à la revendication 19 et un deuxième compartiment contient une composition (B) telle que définie à la revendication 19.

21. Utilisation pour la teinture d'oxydation des fibres kératiniques d'une composition telle que définie dans l'une quelconque des revendications 1 à 17.

## Claims

1. Composition for dyeing keratin fibres, comprising, in a suitable dyeing medium:
• at least one oxidation base chosen from 2,3-diamino-6,7-dihydro-1H,5H-pyrazolo[1,2-a]pyrazol-1-one of formula (I) below, and the addition salts thereof: 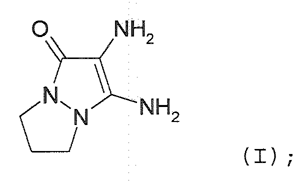
• at least one coupler;
• at least one surfactant chosen from oxyalkylenated or glycerolated fatty alcohols, anionic surfactants of sulfate or sulfonate type and amphoteric surfactants of betaine type; and
• at least one oxidizing agent;
the pH of the composition ranging from 5.5 to 7.5;
with the condition that when the composition comprises an oxyalkylenated or glycerolated fatty alcohol, it is free of 3-amino-2-methylamino-6-methoxypyridine; of fatty-chain cationic polyurethane obtained from the condensation of 1,3-bis(isocyanatomethylcyclohexane), N,N-dimethylethanolamine quaternized with bromodecane, N,N-dimethylethanolamine and polyoxyethylene; of polycondensate of polyethylene glycol containing 150 or 180 mol of ethylene oxide, decyl alcohol and methylenebis(4-cyclohexyl isocyanate); of mixtures of cetearyl alcohol polyglycerolated with 2 mol of glycerol and of cetearyl alcohol polyglycerolated with 6 mol of glycerol; of hexylene glycol.

2. Composition according to Claim 1, in which the oxidation base(s) chosen from 2,3-diamino-6,7-dihydro-1H,5H-pyrazolo[1,2-a]pyrazol-1-one of formula (I) and the addition salts thereof is (are) each present in an amount of between 0.001% and 10% by weight approximately relative to the total weight of the dye composition.

3. Composition according to Claim 1 or 2, in which the couplers are chosen from meta-phenylenediamines, meta-aminophenols, meta-diphenols, naphthalene-based couplers and heterocyclic couplers, and the addition salts thereof.

4. Composition according to Claim 3, in which the coupler(s) is (are) chosen from meta-aminophenols.

5. Composition according to Claim 4, in which the meta-aminophenol(s) is (are) chosen from the compounds of formula (II) below, and the addition salts thereof: 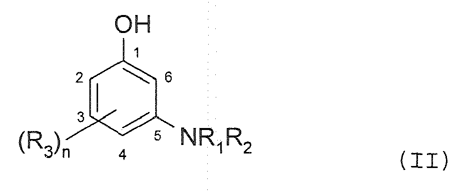 in which:
R₁ and R₂, which may be identical or different, represent a hydrogen atom; an alkyl radical; a monohydroxyalkyl radical; a polyhydroxyalkyl radical; a monoaminoalkyl radical;
R₁ and R₂ form, together with the nitrogen atom to which they are attached, a saturated or unsaturated, 5- to 7-membered cyclic group containing one or more heteroatoms, which may be unsubstituted or substituted with one or more radicals chosen from carboxyl, carboxamido, hydroxyl, amino, monoalkylamino and dialkylamino radicals, and alkyl radicals optionally substituted with one or more hydroxyl, amino, monoalkylamino or dialkylamino radicals;
R₃ represent, independently of each other, a halogen atom; an alkyl radical; an alkoxy radical; a monohydroxyalkyl radical; a polyhydroxyalkyl radical; a monohydroxyalkoxy radical; a polyhydroxyalkoxy radical;
n is an integer between 0 and 3.

6. Composition according to Claim 5, in which R₁ and R₂ denote, independently of each other, a hydrogen atom or a mono- or polyhydroxyalkyl radical.

7. Composition according to any one of the preceding claims, in which the coupler(s) is (are) chosen from chlorinated meta-aminophenols.

8. Composition according to any one of the preceding claims, in which the coupler(s) is (are) each present in an amount of between 0.001% and 10% by weight approximately relative to the total weight of the dye composition.

9. Composition according to any one of the preceding claims, in which the oxyalkylenated fatty alcohol(s) are linear or branched, saturated or unsaturated and comprise 8 to 40 carbon atoms and 1 to 250 ethylene oxide and/or propylene oxide groups.

10. Composition according to Claim 9, in which the oxyalkylenated fatty alcohol(s) are linear or branched, saturated or unsaturated, and comprise 10 to 20 carbon atoms and 2 to 40 ethylene oxide groups.

11. Composition according to any one of the preceding claims, in which the glycerolated fatty alcohol(s) are linear or branched, saturated or unsaturated, and comprise 8 to 40 carbon atoms and 1 to 30 glycerol groups.

12. Composition according to any one of the preceding claims, in which the anionic surfactant(s) of sulfate or sulfonate type are chosen from the salts of alkyl sulfates, alkylamide sulfates, alkyl ether sulfates, alkylamido ether sulfates, alkylaryl ether sulfates, alkyl ether sulfosuccinates, acyl isethionates, methyl acyl taurates and α-olefin sulfonates; the alkyl or acyl radical of all these various compounds comprising from 8 to 24 carbon atoms, and the aryl radical denoting a phenyl or benzyl group; the average number of ethylene oxide or propylene oxide groups ranging from 2 to 50.

13. Composition according to any one of the preceding claims, in which the amphoteric surfactant(s) of betaine type are chosen from (C₈-C₂₀)alkyl betaines, sulfobetaines, (C₈ - C₂₀) alkylamido(C₁-C₆) alkyl betaines and (C₈-C₂₀) alkylamido (C₁-C₆) alkyl sulfobetaines.

14. Composition according to any one of the preceding claims, in which the surfactant(s) chosen from oxyalkylenated or glycerolated fatty alcohols, anionic surfactants of sulfate or sulfonate type and amphoteric surfactants of betaine type represent from 0.05% to 50% by weight relative to the total weight of the composition.

15. Composition according to any one of the preceding claims, comprising at least one additional oxidation base chosen from para-phenylenediamines, bis(phenyl)-alkylenediamines, para-aminophenols, bis-para-aminophenols, 1 ortho-aminophenols, ortho-phenylenediamines and heterocyclic bases other than 2,3-diamino-6,7-dihydro-1H,5H-pyrazolo [1,2-a] pyrazol-1-one, and the addition salts thereof.

16. Composition according to any one of the preceding claims, in which the oxidizing agent(s) is (are) chosen from hydrogen peroxide, urea peroxide, alkali metal bromates, persalts, peracids and oxidase enzymes.

17. Composition according to any one of the preceding claims, in which the oxidizing agent(s) is (are) present in an amount of between 0.01% and 30% by weight relative to the total weight of the composition.

18. Process for dyeing keratin fibres, in which the composition as defined in any one of Claims 1 to 17 is applied to the fibres for a time that is sufficient to develop the desired coloration, after which the fibres are rinsed, optionally washed with shampoo, rinsed again and dried.

19. Process according to Claim 18, comprising a preliminary step that consists in separately storing, on the one hand, a composition (A) comprising, in a suitable dyeing medium, at least one oxidation base chosen from 2,3-diamino-6,7-dihydro-1H,5H-pyrazolo[1,2-a]pyrazol-1-one and the addition salts thereof, at least one coupler and at least one surfactant chosen from oxyalkylenated or glycerolated fatty alcohols, anionic surfactants of sulfate or sulfonate type and amphoteric surfactants of betaine type, and, on the other hand, a composition (B) containing, in a suitable dyeing medium, at least one oxidizing agent, and then in mixing them together at the time of use before applying this mixture to the keratin fibres.

20. Multi-compartment device, in which a first compartment contains a composition (A) as defined in Claim 19 and a second compartment contains a composition (B) as defined in Claim 19.

21. Use, for the oxidation dyeing of keratin fibres, of a composition as defined in any one of Claims 1 to 17.

## Patentansprüche

1. Zusammensetzung zum Färben von Keratinfasern, umfassend in einem für die Färbung geeigneten Medium:
• mindestens eine Oxidationsbase, die unter 2,3-Diamino-6,7-dihydro-1H,5H-pyrazolo[1,2-a]pyrazol-1-on der folgenden Formel (I) und dessen Additionssalzen ausgewählt ist: 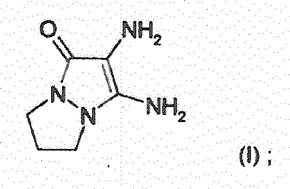
• mindestens einen Kuppler;
• mindestens ein Tensid, das unter oxyalkylenierten oder glycerinierten Fettalkoholen, anionischen Tensiden vom Sulfat- oder Sulfonat-Typ und amphoteren Tensiden vom Betain-Typ ausgewählt ist; und
• mindestens ein Oxidationsmittel;
wobei der pH-Wert der Zusammensetzung im Bereich von 5,5 bis 7,5 liegt;
mit der Maßgabe, dass die Zusammensetzung dann, wenn sie einen oxyalkylenierten oder glycerinierten Fettalkohol umfasst, frei von 3-Amino-2-methylamino-6-methoxypyridin; aus der Kondensation von 1,3-Bis(isocyanatomethylcyclohexan), mit Bromdecan quaternisiertem N,N-Dimethylethanolamin, N,N-Dimethylethanolamin und Polyoxyethylen erhaltenem kationischem Fettketten-Polyurethan; Polykondensat von Polyethylenglykol mit 150 bis 180 mol Ethylenoxid, Decylalkohol und Methylenbis(4-cyclohexylisocyanat); Mischungen von mit 2 mol Glycerin glyceriniertem Cetearylalkohol und mit 6 mol Glycerin glyceriniertem Cetearylalkohol und Hexylenglykol ist.

2. Zusammensetzung nach Anspruch 1, in der die Oxidationsbase bzw. die Oxidationsbasen, die unter 2,3-Diamino-6,7-dihydro-1H,5H-pyrazolo[1,2-a]-pyrazol-1-on der Formel (I) und dessen Additionssalzen ausgewählt sind, jeweils in einer Menge zwischen etwa 0,001 und 10 Gew.-%, bezogen auf das Gesamtgewicht der Färbezusammensetzung, vorliegen.

3. Zusammensetzung nach Anspruch 1 oder 2, in der der Kuppler bzw. die Kuppler unter meta-Phenylendiaminen, meta-Aminophenolen, meta-Diphenolen, Naphthalin-Kupplern, heterocyclischen Kupplern und Additionssalzen davon ausgewählt sind.

4. Zusammensetzung nach Anspruch 3, in der der Kuppler bzw. die Kuppler unter meta-Aminophenolen ausgewählt sind.

5. Zusammensetzung nach Anspruch 4, in der das meta-Aminophenol bzw. die meta-Aminophenole unter den Verbindungen der folgenden Formel (II) und Additionssalzen davon ausgewählt sind: 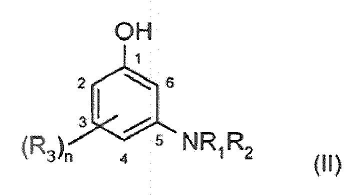 worin:
R₁ und R₂ gleich oder verschieden sind und für ein Wasserstoffatom, einen Alkylrest, einen Monohydroxyalkylrest, einen Polyhydroxyalkylrest oder einen Monoaminoalkylrest stehen;
R₁ und R₂ miteinander und mit dem Stickstoffatom, an das sie gebunden sind, eine gesättigte oder ungesättigte, 5- bis 7-gliedrige cyclische Gruppe, die ein oder mehrere Heteroatome enthält und gegebenenfalls durch einen oder mehrere unter Carboxy-, Carboxamido-, Hydroxyl-, Amino-, Monooder Dialkylaminoresten und gegebenenfalls durch einen oder mehrere Hydroxyl-, Amino-, Mono- oder Dialkylaminorest substituierten Alkylresten ausgewählte Reste substituiert ist, bilden können; die Gruppen R₃ unabhängig voneinander für ein Halogenatom, einen Alkylrest, einen Alkoxyrest, einen Monohydroxyalkylrest, einen Polyhydroxyalkylrest, einen Monohydroxyalkoxyrest oder einen Polyhydroxyalkoxyrest stehen und
n für eine ganze Zahl zwischen 0 und 3 steht.

6. Zusammensetzung nach Anspruch 5, in der R₁ und R₂ unabhängig voneinander für ein Wasserstoffatom oder einen Mono- oder Polyhydroxyalkylrest stehen.

7. Zusammensetzung nach einem der vorhergehenden Ansprüche, in der der Kuppler bzw. die Kuppler unter chlorierten meta-Aminophenolen ausgewählt sind.

8. Zusammensetzung nach einem der vorhergehenden Ansprüche, in der der Kuppler bzw. die Kuppler jeweils in einer Menge zwischen etwa 0,001 und 10 Gew.-%, bezogen auf das Gesamtgewicht der Färbezusammensetzung, vorliegen.

9. Zusammensetzung nach einem der vorhergehenden Ansprüche, in der der oxyalkylenierte Fettalkohol bzw. die oxyalkylenierten Fettalkohole linear oder verzweigt und gesättigt oder ungesättigt sind und 8 bis 40 Kohlenstoffatome und 1 bis 250 Ethylenoxid- und/oder Propylenoxidgruppen enthalten.

10. Zusammensetzung nach Anspruch 9, in der der oxyalkylenierte Fettalkohol bzw. die oxyalkylenierten Fettalkohole linear oder verzweigt und gesättigt oder ungesättigt sind und 10 bis 20 Kohlenstoffatome und 2 bis 40 Ethylenoxidgruppen enthalten.

11. Zusammensetzung nach einem der vorhergehenden Ansprüche, in der der glycerinierte Fettalkohol bzw. die glycerinierten Fettalkohole linear oder verzweigt und gesättigt oder ungesättigt sind und 8 bis 40 Kohlenstoffatome und 1 bis 30 Glyceringruppen enthalten.

12. Zusammensetzung nach einem der vorhergehenden Ansprüche, in der das anionische Tensid bzw. die anionischen Tenside vom Sulfat- oder Sulfonat-Typ unter Salzen von Alkylsulfaten, Alkylamidosulfaten, Alkylethersulfaten, Alkylamidoethersulfaten, Alkylarylethersulfaten, Alkylethersulfosuccinaten, Acylisethionaten, Methylacyltauraten und α-Olefinsulfonaten ausgewählt sind; wobei der Alkyl- oder Acylrest aller dieser verschiedenen Verbindungen 8 bis 24 Kohlenstoffatome enthält und der Arylrest für eine Phenyl- oder Benzylgruppe steht; wobei die durchschnittliche Zahl von Ethylenoxid- und/oder Propylenoxidgruppen 2 bis 50 beträgt.

13. Zusammensetzung nach einem der vorhergehenden Ansprüche, in der das amphotere Tensid bzw. die amphoteren Tenside vom Betain-Typ unter (C₈-C₂₀)-Alkylbetainen, Sulfobetainen, (C₈-C₂₀) -Alkylamido-(C₁-C₆) -alkylbetainen oder (C₈-C₂₀) -Alkylamido- (C₁-C₆)-alkylsulfobetainen ausgewählt sind.

14. Zusammensetzung nach einem der vorhergehenden Ansprüche, in der das Tensid bzw. die Tenside, die unter oxyalkylenierten oder glycerinierten Fettalkoholen, anionischen Tensiden vom Sulfat- oder Sulfonat-Typ und amphoteren Tensiden vom Betain-Typ ausgewählt sind, 0,05 bis 50 Gew.-% des Gesamtgewichts der Zusammensetzung ausmachen.

15. Zusammensetzung nach einem der vorhergehenden Ansprüche, umfassend mindestens eine zusätzliche Oxidationsbase, die unter para-Phenylendiaminen, Bisphenylalkylendiaminen, para-Aminophenolen, Bispara-aminophenolen, ortho-Aminophenolen, ortho-Phenylendiaminen, heterocyclischen Basen, die von 2,3-Diamino-6,7-dihydro-1H,5H-pyrazolo[1,2-a]-pyrazol-1-on verschieden sind, sowie Additionssalzen davon ausgewählt ist.

16. Zusammensetzung nach einem der vorhergehenden Ansprüche, in der das Oxidationsmittel bzw. die Oxidationsmittel unter Wasserstoffperoxid, Harnstoffperoxid, Alkalimetallbromaten, Persalzen, Persäuren und Oxida.seenzymen ausgewählt sind.

17. Zusammensetzung nach einem der vorhergehenden Ansprüche, in der das Oxidationsmittel bzw. die Oxidationsmittel in einer Menge zwischen 0,01 und 30 Ges.-%, bezogen auf das Gesamtgewicht der Zusammensetzung, vorliegen.

18. Verfahren zum Färben von Keratinfasern, bei dem man auf die Fasern die Zusammensetzung gemäß einem der Ansprüche 1 bis 17 über einen zur Entwicklung der gewünschten Färbung ausreichenden Zeitraum aufbringt und dann spült, gegebenenfalls mit Shampoo wäscht, erneut spült und trocknet.

19. Verfahren nach Anspruch 18 mit einem vorgeschalteten Schritt, bestehend aus der getrennten Aufbewahrung einerseits einer Zusammensetzung (A), die in einem für die Färbung geeigneten Medium mindestens eine Oxidationsbase, die unter 2,3-Diamino-6,7-dihydro-1H,5H-pyrazolo[1,2-a]pyrazol-1-on und dessen Additionssalzen ausgewählt ist, mindestens einen Kuppler und mindestens ein Tensid, das unter oxyalkylenierten oder glycerinierten Fettalkoholen, anionischen Tensiden vom Sulfat- oder Sulfonat-Typ und amphoteren Tensiden vom Betain-Typ ausgewählt ist, umfasst, und andererseits einer Zusammensetzung (B), die in einem für die Färbung geeigneten Medium mindestens ein Oxidationsmittel enthält, und nachfolgendem Mischen davon zum Zeitpunkt der Anwendung vor dem Aufbringen dieser Mischung auf die Keratinfasern.

20. Vorrichtung mit mehreren Kompartimenten, in der ein erstes Kompartiment eine Zusammensetzung (A) gemäß Anspruch 19 enthält und ein zweites Kompartiment eine Zusammensetzung (B) gemäß Anspruch 19 enthält.

21. Verwendung einer Zusammensetzung gemäß einem der Ansprüche 1 bis 17 zum Oxidationsfärben von Keratinfasern.
